# EUROPEAN PATENT SPECIFICATION

(11) **EP 1 052 280 B1**
(45) Date of publication and mention of the grant of the patent: **23.08.2006**
(21) Application number: 99973305.8
(22) Date of filing: 02.12.1999
(51) Int. Cl.: C10G 35/06, C10G 61/00, B01J 27/055, B01J 32/00

(54) **METHOD FOR ISOMERIZATION OF HYDROCARBON, AND SOLID ACID CATALYST AND ISOMERIZATION SYSTEM FOR USE THEREIN**
VERFAHREN ZUR ISOMERISATION VON KOHLENWASSERSTOFFEN, FESTER SÄUREKATALYSATOR UND DAFÜR VERWENDETE VORRICHTUNG
PROCEDE D'ISOMERISATION D'HYDROCARBURES, CATALYSEUR ACIDE SOLIDE ET SYSTEME D'ISOMERISATION UTILISE AVEC CE PROCEDE

(30) Priority: 04.12.1998 JP 34531298; 21.01.1999 JP 1254999; 25.05.1999 JP 14458499
(43) Date of publication of application: 15.11.2000
(73) Proprietor: JAPAN ENERGY CORPORATION, Tokyo 105-0001 (JP)
(72) Inventor: MATSUZAWA, Kenji, Japan Energy Corporation, Toda-shi, Saitama 335-0026 (JP); AIMOTO, Kohjiroh, Japan Energy Corporation, Toda-shi, Saitama 335-0026 (JP)
(74) Representative: Schwabe - Sandmair - Marx
(86) International application number: PCT/JP1999/006769
(87) International publication number: WO 2000/034415

(56) References cited:
- WO-A-98/09727
- WO-A1-92/11344
- JP-A- 4 187 239
- JP-A- 9 075 735
- JP-A- 10 195 001
- US-A- 4 280 899
- US-A- 4 923 595

## Description

### TECHNICAL FIELD

The present invention relates to a method of isomerizing hydrocarbons at a high efficiency using a sulfated zirconia solid acid catalyst, and a solid acid catalyst used for the isomerization and an isomerization device used for the same.

### BACKGROUND ART

Isomerization of hydrocarbons, such as petroleum cuts, etc., is important as a method of producing high-octane-level gasoline base materials and as a method of producing chemical starting materials. Isomerization of normal butane is a particularly important reaction as a method of producing isobutane, which is a starting material for methyl-tert-butyl ether and alkylate gasoline, while isomerization of light naphtha is a particularly important reaction as a method of producing gasoline base material.

Conventional isomerization of hydrocarbons is performed by bringing a catalyst, which has been obtained by modifying an oxide showing solid acid properties with platinum, such as platinum-zeolite, platinum-chlorinated alumina etc., into contact with hydrocarbons in the presence of hydrogen. Reaction catalyzed with the platinum-zeolite catalyst is usually performed using a reaction starting material having a water concentration of several tens ppm and it is not known that its catalytic activity is improved by reducing the water concentration in the reaction starting material. Generally, the catalytic activity of the platinum zeolite catalyst is much lower than that of the platinum-chlorinated alumina. On the other hand, it is known that the activity of the platinum-chlorinated alumina catalyst is reduced by impurities, such as water concentration, etc., in the reaction starting materials, and the reaction is usually performed with the water concentration in the reaction starting materials controlled to be 0.5 ppm or less, particularly 0.1 ppm are less. If the water concentration in the reaction starting material is limited to 0.5 ppm or less, the platinum-chlorinated alumina catalyst will show much higher catalytic activity than platinum-zeolite catalyst does. However, in an isomerization reaction using the platinum-chlorinated alumina catalyst, it was necessary to continuously introduce an organic chlorine compound to the reaction system in order to retain the catalytic activity and the chlorine compound had to be separated and removed from the product after the reaction was completed. Today the use of such a chlorine compound should be avoided in terms of environmental protection, including problems with corrosion during the separation and removal process.

US-A-4,923,595 discloses a method for the reforming of hydrocarbon using a catalyst comprising a refractory support having a nominal diameter of at least 650 microns and having deposited thereon a uniformly dispersed platinum component, a uniformly dispersed IV metal component and a surface-impregnated metal or metal-containing component selected from the group consisting of rhodium, ruthenium, cobalt, nickel or iridium. Reforming conditions utilised include a pressure selected from the range of 101 to 6995 kPa and a temperature selected from the range of 427 to 593 °C. The preferred carrier materials of the catalyst are refractory inorganic oxides such as zirconium dioxide onto which chlorine is introduced.

### DISCLOSURE OF THE INVENTION

Consequently, there is a demand for a catalyst of high activity with which it is not necessary to introduce a chlorine compound to the process of isomerization of hydrocarbons. An object of the present invention is to solve this problem and provide a method of isomerizing hydrocarbons at a high efficiency without using a chlorine compound, as well as a catalyst used for this method and an isomerization device for executing this method.

As the result of intense studies, the inventors completed the present invention upon discovering that when a solid acid catalyst comprising a support having portions made of zirconia and/or hydrated zirconia having a tetragonal crystal structure, and a sulfureous component supported in an amount of 1 to 10 wt% in terms of the amount of sulfur in the solid acid catalyst on this support is used, the catalytic activity of the catalyst is improved by reducing the water concentration in hydrocarbons, which are the reaction starting material, to 5 ppm or less, particularly 1 ppm or less, and isomerization of the hydrocarbons can be performed with a high efficiency without introducing any chlorine compound to the reaction system.

More particularly, in the method of isomerization of hydrocarbons of the present invention as defined in claim 1, a solid acid catalyst comprising a support having portions made of zirconia and/or hydrated zirconia, and a sulfureous component supported on this support in an amount of 1 to 10 wt%, in terms of the amount of sulfur in the solid acid catalyst, is brought into contact with starting hydrocarbons as a raw material having a water concentration of 5 ppm or less, preferably 1 ppm or less, in terms of the weight of the water concentration, in the presence of hydrogen to obtain isomerized hydrocarbons and carrying out the isomerization under the conditions of claim 1. It is preferred that the solid acid catalyst preferably comprise at least one metal component selected from the group consisting of Group 8, Group 9 and Group 10 metals and/or the support comprise portions made of zirconia and/or hydrated zirconia and portions made of alumina and/or hydrated alumina.

In the present invention, the water concentration to be concerned is an amount of the water contained in the reaction system. Hydrogen, which is one of the starting materials, is usually readily obtained with water concentration of 0.5 ppm or less. Therefore, the water concentration to be really concerned is that in the starting hydrocarbons. According to a second aspect the present invention pertains to a hydrocarbon isomerization device, comprising:
a dehydration device, which reduces water concentrations of starting hydrogen and starting hydrocarbons;
a catalytic reactor, to which the starting hydrogen and the starting hydrocarbons that have passed through said dehydration device and recycled hydrogen are fed and in which a solid acid catalyst comprising a support which comprises portions made of zirconia and/or hydrated zirconia having a tetragonal crystal structure, and a sulfureous component supported in an amount of 1 to 10wt%, in terms of the amount of sulfur in the solid acid catalyst, on the support is charged;
a separation device, which separates a reaction product forwarded from the catalytic reactor into hydrogen to be recycled and a hydrocarbon product; and
a compressor, which compresses the recycled hydrogen forwarded from the separation device and feeds it to the catalytic reactor.

### BRIEF DESCRIPTION OF THE DRAWINGS

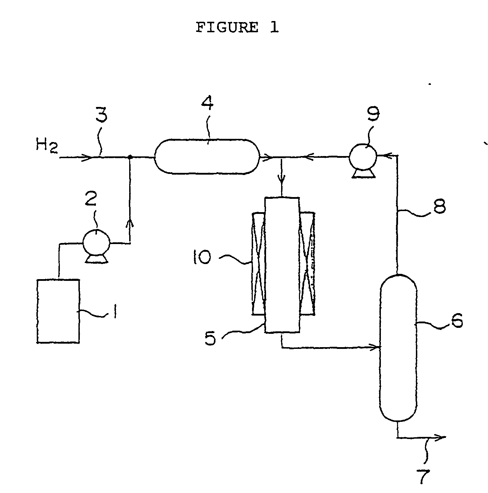

Figure 1 is a diagram showing a preferred example of an isomerization device for performing the method of isomerization of hydrocarbons of the present invention. In Figure 1, 1 is a starting material tank, 2 is a pump, 3 is a hydrogen line, 4 is a dehydration device, 5 is a catalytic reactor, 6 is a gas-liquid separation device, 7 is a lower piping, 8 is an upper piping, 9 is the compressor, and 10 is a electric heater.

### BEST MODE FOR CARRYING OUT THE INVENTION

### [Starting hydrocarbons]

Light naphtha or a whole naphtha obtained by distillation and separation of crude oil, butane obtained from a fluid catalytic cracker, etc., can be used as the starting hydrocarbons that are the subject of isomerization of the present invention. A single hydrocarbon compound or a mixture of several hydrocarbon compounds with a boiling point range of preferably -20°C to 120°C, particularly a boiling point range of -5°C to 110°C, is preferred. Furthermore, the starting hydrocarbons contain a water concentration of 5 ppm or less, particularly 1 ppm or less, further preferably 0.5 ppm or less, in terms of weight of the water concentration. Hydrocarbons containing a water concentration exceeding this range are used after reducing the water concentration with an adsorbent, etc.

### [Isomerization reaction conditions]

The isomerization reaction conditions of the present invention vary with the catalyst used and the purpose of the reaction, but usually the reaction temperature range is 50°C to 250°C, more preferably 100°C to 200°C, the reaction pressure range is 1.01-5.050 kPa (1 to 50 kgf/cm²), more preferably 1,515-4,040 kPa (15 to 40 kgf/cm²), and the preferred liquid hourly space velocity LHSV range is 0.2 to 10/hr. The range of the hydrogen/(hydrocarbons in the starting material) ratio is preferably not less than the amount of hydrogen necessary for saturation of the unsaturated component (olefin component, aromatic component, etc.) contained in the starting hydrocarbons, more preferably not less than 0.1 mol/mol and not more than 10 mol/mol.

The solid acid catalyst used before the isomerization reaction can be subjected to a reduction treatment with hydrogen. The treatment temperature in this case is preferably a temperature lower than 300°C, particularly preferably a temperature lower than 250°C. If the treatment temperature is too high, the sulfureous component in the catalyst will be reduced and there will be a deterioration in catalytic activity. However, this reduction treatment is not essential and it is not particularly necessary to carry out reduction treatment if the reaction is performed in a hydrogen ambient atmosphere. Moreover, the catalyst may be treated with an oxidizing gas, such as air, etc., prior to the reduction.

### [Impurities in starting hydrocarbons]

In the isomerization method of the present invention, the concentration of the water contained in the hydrocarbons serving as the starting material is 5 ppm by weight or less, desirably 1 ppm by weight or less, more desirably 0.5 ppm by weight or less. The water contained in the hydrocarbons can be analyzed with a Karl Fischer moisture meter, etc. The concentration of sulfur compounds contained in the hydrocarbons serving as the starting material is desirably 5 ppm by weight or less, more desirably 1 ppm by weight or less, further desirably 0.5 ppm by weight or less, in terms of sulfur. The sulfur compounds contained in the hydrocarbons can be analyzed by a gas chromatograph equipped with an atomic emission detector (AED), etc. As for the concentrations of other impurities in the hydrocarbons as the starting material, the concentration of nitrogen compounds is desirably 5 ppm by weight or less, more desirably 1 ppm by weight or less, particularly desirably 0.1 ppm by weight or less, in terms of nitrogen. The concentration of oxygen compounds other than water is desirably 5 ppm by weight or less, more desirably 1 ppm by weight or less, particularly desirably 0.1 ppm by weight or less, in terms of oxygen. It is advisable that the concentration of arsenic compounds be 5 ppb by weight or less in terms of arsenic. It is advisable that the concentration of lead compounds be 20 ppb by weight or less in terms of lead. It is advisable that the concentration of copper compounds be 20 ppb by weight or less in terms of copper. The concentration of olefins is preferably 5 wt% or less, more preferably 1 wt% or less. The concentration of chlorine compounds is preferably 1 ppm by weight or less, more preferably 0.1 ppm by weight or less, in terms of chlorine.

### [Impurities in hydrogen]

As for the concentration of impurities in the hydrogen used by the isomerization method of the present invention, the water concentration is desirably 5 ppm by weight or less, particularly desirably 1 ppm by weight or less, further desirably 0.5 ppm by weight or less. The concentration of sulfur compounds is desirably 10 ppm by weight or less, more desirably 5 ppm by weight or less, particularly desirably 1 ppm by weight or less, in terms of sulfur. The concentration of nitrogen compounds is desirably 50 ppm by weight or less, more desirably 10 ppm by weight or less, particularly desirably 1 ppm by weight or less, in terms of nitrogen. The concentration of oxygen compounds other than water is desirably 500 ppm by weight or less, more desirably 100 ppm by weight or less, particularly desirably 50 ppm by weight or less, in terms of oxygen.

### [Solid acid catalyst]

A conventional solid acid catalyst known as a sulfated zirconia system (solid acid catalyst comprising a support, which comprises portions made of zirconia and/or hydrated zirconia having a tetragonal crystal structure, and a sulfureous component supported in an amount of 1 to 10 wt%, in terms of the amount of sulfur in the solid acid catalyst, on this support) can be used as the solid acid catalyst in the present invention. Furthermore, as long as the effects contemplated by the present invention are obtained, the portions made of zirconia and/or hydrated zirconia may also contain other metal components. Titanium, hafnium, vanadium, chromium, manganese, iron, silicon, gallium, etc., can be as the other metal components. These metal components can be used in the form of a composite oxide and/or a hydrated composite oxide which may be mixed with a further component of a single oxide and/or a hydrated oxide. It is preferred that the amount of the portions made of zirconia and/or hydrated zirconia accounts for 20 to 99 wt%, particularly 50 to 99 wt%, of the catalyst weight.

The concentration of the sulfureous component of the solid acid catalyst is 1 to 10wt%, in terms of the amount of sulfur. Platinum, palladium, ruthenium, nickel, etc., are particularly ideal for use as the metal component selected from Group 8, Group 9, and Group 10, and it is preferred that they be added so that the total amount of the metal component of Group 8, Group 9 and/or Group 10 accounts for 0.05 to 10 wt% of the solid acid catalyst.

The crystal structure of the portions consisting of zirconia and/or hydrated zirconia of the catalyst used in the present invention is preferably a tetragonal system. A monoclinic crystal structure can also be present in part. These crystal structures can be confirmed by X-ray analysis. In concrete terms, the area ratio of the peak at 2θ = 28.2° to that at 2θ = 30.2°C determined by X-ray diffractometry with CuKα rays (hereinafter, abbreviated as "S28.2/S30.2 ratio" wherein S28.2 is the area of the peak of monoclinic zirconia at 2θ = 28.2° and S30.2 is the area of the peak of tetragonal zirconia at 2θ = 30.2°) is 1.0 or less, preferably 0.3 or less, particularly preferably 0.05 or less. Higher catalytic activity is obtained when there is almost no monoclinic structure present.

### [Preferred solid acid catalyst]

In the isomerization reaction of the present invention, it is preferred that a catalyst comprising a support comprising portions made of zirconia and/or hydrated zirconia having a tetragonal crystal structure (hereafter, also referred to simply as "zirconia") and portions made of alumina and/or hydrated alumina (hereafter, also referred to simply as "alumina"), a sulfureous component supported in an amount of 1 to 10 wt%, in terms of the amount of sulfur in the solid acid catalyst on this support, and the metal component selected from among the metals of Group 8, Group 9 and or Group 10 be used. It is preferred that this catalyst be made by kneading and shaping aluminum hydroxide and/or hydrated aluminum oxide, zirconium hydroxide and/or hydrated zirconium oxide, and a sulfur-containing compound, calcining the shaped article thus obtained at a temperature at which zirconia with a tetragonal crystal structure is obtained, supporting the metal component of selected from Group 8, Group 9, or Group 10, and then calcining at a temperature of 300 to 700°C.

When producing the catalyst that is the subject of the present invention, the aluminum hydroxide and/or hydrated aluminum oxide to be used can be obtained by a variety of methods. If α-alumina and γ-alumina, which are aluminum oxides, are used in place of the aluminum hydroxide and/or hydrated aluminum oxide, there will be a reduction in crushing strength of the catalyst, and monoclinic zirconia will readily enters into the catalyst during calcination following shaping, leading to a reduction in catalytic activity.

With respect to the aluminum hydroxide and/or hydrated aluminum oxide, the weight of alumina accounts for 5 to 90 wt%, preferably 5 to 50 wt%, particularly preferably 10 to 50 wt%, of the total weight of the alumina and zirconia in the catalyst. If it is less than this range, there will be a reduction in the crushing strength of the catalyst and it will be difficult to stabilize the zirconia. If it exceeds this range, there will be a relative reduction in catalytic activity.

The aluminum hydroxide and/or hydrated aluminum oxide is generally used in the form of a powder with an average particle size of preferably 0.5 to 50 µm, more preferably 0.5 to 20 µm, in order to improve the catalytic activity and crushing strength. The use of a hydrated aluminum oxide with a boehmite structure, such as pseudoboehmite, etc., as the aluminum hydroxide and/or hydrated aluminum oxide can improve the catalytic activity and therefore is preferred.

Although the hydroxide and/or hydrated oxide of zirconium can be prepared by any method, in general they are made by neutralization or hydrolysis of their salts and organometallic compounds, such as oxychlorides or alcoholates, chlorides, sulfates, nitrates, oxysulfates, etc. The catalyst crushing strength is improved and the zirconia is easily stabilized when the hydroxide or hydrated oxide of zirconium is amorphous, having no obvious crystal structure by X-ray or electron beam diffraction. Moreover, it is usually preferred that zirconium hydroxide and/or hydrated zirconium oxide in the form of a powder with a mean particle size of 0.5 to 50µm, particularly 0.5 to 20 µm, be used in order to improve catalytic activity and crushing strength.

The sulfur-containing compound is a compound containing a sulfureous component or a sulfur-containing compound that can later be converted to a sulfureous component by a treatment such as calcination, etc.

Sulfuric acid, ammonium sulfate, sulfurous acid, ammonium sulfite, thionyl chloride, etc., are given as examples of the sulfur-containing compounds. However, sulfuric acid-containing compounds are preferably used, and ammonium sulfate and dimethyl sulfate are preferred because of their low corrosivity to the production equipment. The sulfur-containing compound can be used as is, or it can be used in the form of a solution, such as an aqueous solution.

With respect to the weight of the sulfur-containing compound mixed during the production of the catalyst, preferably 3 to 40 wt%, more preferably 10 to 30 wt% of the total weight of aluminum hydroxide and/or hydrated aluminum oxide, zirconium hydroxide and/or hydrated zirconium oxide, and sulfur-containing compound is preferred because the catalytic activity is improved. It is particularly preferred that ammonium sulfate, etc., be used as the sulfur-containing compound in a solid form.

Furthermore, the sulfur-containing compound can be in a solid form or liquid form. Moreover, there are no particular restrictions to the concentration of the solution and it can be prepared taking into consideration the amount of solution that it is necessary for the kneading that will be performed after. The amount of sulfur-containing compound added is preferred such that the amount of sulfur accounts for 1 to 10 wt%, of the finally obtained solid acid catalyst. There are no particular restrictions to the mixing method.

Any type of kneading machine can be used for kneading during the production of the catalyst as long as it is a kneading machine generally used for catalyst preparation. Usually, a method wherein after the starting materials are introduced to a kneading machine, water is added and the materials are mixed with mixing blades is preferably used, but there are no particular restrictions to the order of introduction of starting materials and additives, etc. Water is normally added during kneading, but it is not necessary to add water when powder in a slurry form is used, etc. An organic solvent, such as ethanol, isopropanol, acetone, methyl ethyl ketone, methyl isobutyl ketone, etc., can also be used as a liquid other than water that is added. The temperature and time during kneading can be appropriately selected depending on the properties of the aluminum hydroxide and/or hydrated aluminum oxide, zirconium hydroxide and/or hydrated zirconium oxide, and sulfur-containing compound used as the starting materials, etc., and there are no particular restrictions as long as these conditions will not significantly affect the intended catalyst performance. Moreover, acid, such as nitric acid, etc., and base, such as ammonium, etc., organic compounds, binder, ceramic fibers, surfactant, zeolite, etc., can be added and kneaded as long as similarly within a range with which catalyst performance of the present invention are ensured. However, the catalyst of the present invention has sufficient strength and high catalytic activity, even if such additives are added during kneading.

Any shaping method generally used for catalyst preparation can be employed for shaping after kneading during catalyst preparation. Extrusion shaping using a screw-type extruder, etc., is preferably used because shaping into any shape, such as pellets, honeycomb shape, etc., with good efficiency is possible. There are no particular restrictions to the size of shaped article, but it is usually shaped to a length in cross section of 0.2 mm or longer, preferably 0.5 to 20 mm. For instance, cylindrical pellets with a diameter of 0.5 to 10 mm and a length of 0.5 to 15 mm can usually easily be obtained. Crushing strength after calcination is strongly affected by kneading and therefore, pre-determination is advisable for the water concentration, kneading time, the amount of electricity, etc., during the above-mentioned kneading.

The operation is simple, having great industrial merit, because there are no processes for filtration and drying, etc., between the step of mixing and kneading of the sulfur-containing compound with other materials and the shaping step. Moreover, because a shaped catalyst is obtained, it is ideal for fixed bed reactions, which are difficult with conventional powder catalysts.

Calcination after shaping is conducted at a temperature, at which tetragonal zirconium oxide can be obtained, in air or a gas ambient atmosphere, such as nitrogen, etc. When pseudoboehmite-type aluminum is used, the preferred calcination temperature is 450 to 800°C, particularly 500 to 800°C, further 600 to 800°C. Moreover, the preferred calcination time is 0.1 to 20 hours. There are cases where the proportion of monoclinic crystals in the zirconium oxide crystals will increase and the peak area ratio of the peak at 2 2θ = 28.2° to that at 2θ = 30.2° will exceed 1, and the catalytic activity will also drop, when the calcination temperature is too high, making this undesirable. Moreover, the zirconium oxide will not crystallize and there will also be a drop in catalytic activity if the calcination temperature is too low, making this undesirable.

Platinum group metals, such as platinum, palladium, ruthenium, etc., are particularly ideal as the metal component selected from Group 8, Group 9, and Group 10 contained in the catalyst used in the present invention. It is preferred that these be used in the form of a compound rather than as the metal itself. These metal compounds can be anhydrides or hydrates. Furthermore, these metal compounds may be used either singly or in a combination of two or more thereof. It is preferred that these metal compounds be added in an amount such that the total amount of Group 8, Group 9, and Group 10 elements accounts for 0.05 to 10 wt% of the finally obtained solid acid catalyst.

Although there are no particular restrictions to the supporting method, an impregnation method such as spraying or immersion, etc., ion exchange methods, etc., are ideal. Calcination of the above-mentioned supported articles for 0.1 to 20 hours at a temperature higher than 300°C and lower than 700°C in air or a gas ambient atmosphere, such as nitrogen, etc., is preferred because catalytic activity will be increased.

It is preferred that the total amount of zirconia and alumina in the catalyst of the present invention be brought to 70 wt% or more, particularly 80 wt% or more, in terms of catalytic activity, strength of the shaped article, etc. For practical use, it is preferred that the shaping strength of the catalyst be 3 kg or more in terms of radial crushing strength of cylindrical pellets with a diameter of 1.5 mm.

Next, the preferred isomerization device for executing the method of isomerization of hydrocarbons of the present invention will now be described.

### [Isomerization device]

The isomerization device that is one aspect of the present invention comprises:
a dehydration device, which reduces the water concentration of the starting hydrogen and the starting hydrocarbons;
a catalytic reactor, to which the starting hydrogen and the starting hydrocarbons that have passed through the dehydration device and recycled hydrogen are fed and in which a solid acid catalyst comprising a support, which comprises portions made of zirconia and/or hydrated zirconia having a tetragonal crystal structure, and a sulfureous component supported in an amount of 1 to 10 wt.%, in terms of the amount of sulfur in the solid acid catalyst on this support is introduced;
a separation device, which separates the product from the catalytic reactor into hydrogen to be recycled and the hydrocarbon product; and
a compressor, which compresses the recycled hydrogen forwarded from the separation device and feeds it to the catalytic reactor.

The structure of a typical isomerization device is shown in Figure 1. Starting hydrocarbons are fed from a starting material tank 1 by a pump 2, while starting hydrogen is introduced from a hydrogen line 3, with these starting materials two being mixed inside the piping and introduced to a dehydration device 4. The starting material that has passed through the dehydration device 4 is introduced to a catalytic reactor 5 charged with the above-mentioned solid acid catalyst. The product in the catalytic reactor 5 is introduced to a gas-liquid separation device 6 and the hydrocarbon product is emitted from a lower piping 7. The unreacted hydrogen that has been separated by the gas-liquid separation device 6 is emitted from an upper piping 8 and compressed by a compressor 9, mixed with the starting material that has passed through the dehydration device 4, and introduced once again to the catalytic reactor 5. MOLECULAR SIEVES 3A has been packed in the dehydration device 4.

### [Dehydration device]

The dehydration device reduces the water concentration of the starting hydrocarbons and, when necessary, the starting hydrogen. In particular, the former usually contains a water concentration exceeding 5 ppm and, therefore, the water concentration is reduced to 5 ppm by weight or less, particularly preferably 1 ppm by weight or less, further preferably 0.5 ppm by weight or less, by the dehydration process. A generally well-known method, for instance, a method using an adsorbent such as MOLECULAR SIEVES 3A or alumina, distillation, etc., can be used for the dehydration process, but it is not limited to these methods. Any type of method can be used as long as it is possible to bring the water concentration of the starting materials to the above-mentioned water concentration in terms of weight or lower, but a method using an adsorbent is preferably used. The starting hydrocarbons and, when necessary, the starting hydrogen can also be the dehydrated by separate devices, but it is preferred that a mixture of the two be dehydrated. Furthermore, the water concentration included in the hydrocarbons can be analyzed by a Karl Fischer moisture meter, etc.

It is also preferred that the sulfur concentration, nitrogen concentration, and oxygen concentration other than water be reduced. Specifically, the concentration of sulfur compounds is desirably 5 ppm by weight or less, preferably, more desirably 1 ppm by weight or less, further desirably 0.5 ppm by weight or less, in terms of sulfur. The concentration of nitrogen compounds is desirably 5 ppm by weight or less, more desirably 1 ppm by weight or less, further desirably 0.1 ppm by weight or less, in terms of nitrogen. The concentration of oxygen compounds other than water is desirably 5 ppm by weight or less, more desirably 1 ppm by weight or less, further desirably 0.1 ppm by weight or less, in terms of oxygen.

### [Catalytic reactor]

A fixed-bed reactor is generally used for isomerization, but a flowing-bed or moving-bed reactor can also be used. Moreover, the direction of flow of the reaction starting materials in the catalyst layer can be in any direction, including from above to below the catalyst layer, from the below to above the catalyst layer, and from the center of the catalyst layer to the outside of the catalyst layer. Recycled hydrogen and starting hydrocarbons and starting hydrogen that have been passed to the dehydration device are fed to the catalytic reactor. These are normally mixed and then fed, but they can also be fed from separate inlets into the reactor. The starting materials that will be fed to the catalytic reactor are usually heated after dehydration in order to bring their temperature to the temperature appropriate for isomerization, but the catalytic reactor itself can also have a heating means.

### [Separation device]

According to the present invention, a separation device is provided for separating the product forwarded from the catalytic reactor into hydrogen to be recycled and hydrocarbons. The hydrogen and the hydrocarbon product can be separated based on the difference in their boiling points, and they are usually separated by means of gas-liquid separation. However, they can also be separated by distillation, etc. In addition to isomerized hydrocarbons, the hydrocarbon product comprises a component that is starting hydrocarbon residue, by-product resulting from decomposition of the hydrocarbons, etc., and these can also be simultaneously separated by the separation device.

The straight-chain or mono-branched hydrocarbons included in the hydrocarbons from which hydrogen has been separated, are further separated from the product, mixed with starting hydrocarbon, and re-reacted and as a result, it is also possible to further improve the ratio of isomers in the product. A separation technique using a zeolite adsorbent having a shape selectivity or separation by means of distillation can be used as for separation of the straight-chain hydrocarbons, etc. from the product

### [Compressor]

In the present invention, a compressor is used for compressing the recycled hydrogen forwarded from the separation device to the necessary pressure required in the catalytic reactor. There are no particular restrictions to the structure of the compressor as long as the necessary pressure is obtained.

### Examples

The present invention will now be described in detail with examples.

### Example 1

### [Method of determining mean particle size]

The mean particle size was determined by a wet determination method using MICROTRAC particle size analyzer made by Nikkiso K.K. This involves irradiating flowing powder with a laser beam and analyzing the particle size based on the forward scattered light.

### [Method of preparation of solid acid catalyst]

Commercial zirconium hydroxide was dried to obtain dry hydrated zirconium with a mean particle size of 1.2 µm. Then, 500 g of hydrated alumina (pseudo-boehmite powder) with a mean particle size of 10 µm was added to 1.50 kg of the dry hydrated zirconia powder. Next, 383 g of ammonium sulfate was further added and [the mixture] was kneaded for 45 minutes with a kneading machine with mixing blades while adding water. The kneaded product thus obtained was extruded from an extruder with a round orifice (diameter of 1.6 mm) and dried at 110°C to obtain dry pellets. Then, the dry pellets were calcined for 2 hours at 650°C to obtain a zirconia shaped article supporting a sulfureous component thereon. Next, 125 ml of an aqueous solution of chloroplatinic acid to bring the amount of platinum in the catalyst to 0.5% was added to 50 g of the zirconia shaped article. After this was dried, it was calcined for 2 hours at 500°C to obtain a solid acid catalyst that was a zirconia/aluminum shaped catalyst supporting platinum and a sulfureous component.

### [Reaction starting materials]

Light naphtha was used as a hydrocarbon starting material for isomerization. This light naphtha comprised 4.9 wt% butane, 56.3 wt% pentane, 32.6 wt% hexane, 2.3 wt% cyclopentane, 2.8 wt% methyl cyclopentane and cyclohexane, 1.1 wt% benzene, and 0.1 wt% olefin. The light naptha had a water concentration of 40 ppm by weight and a sulfur concentration of not more than 1 ppm by weight. This light naphtha served as reaction starting material 1. The amount of hydrogen needed for hydrogenation of the unsaturated component in this light naphtha was 0.03 mol per 1 mol light naphtha. Moreover, the water concentration of the light naphtha as the reaction starting material had been brought to 20 ppm by weight, 0.9 ppm by weight, 0.4 ppm by weight, and 0.1 ppm by weight by dehydration with MOLECULAR SIEVES 3A to provide reaction starting material 2, reaction starting material 3, reaction starting material 4, and reaction starting material 5, respectively. As for the other impurities included in the reaction starting materials 1 to 5, the concentration of sulfur compounds was 1 ppm by weight or less in terms of sulfur, the concentration of nitrogen compounds was 0.1 ppm by weight or less in terms of nitrogen, the concentration of oxygen compounds other than water was 0.1 ppm by weight or less in terms of oxygen, the concentration of arsenic compounds was 5 ppb by weight or less in terms of arsenic, the concentration of lead compound was 20 ppb by weight or less in terms of lead, the concentration of copper compounds was 20 ppb by weight or less in terms of copper, and the concentration of chlorine compounds was 0.1 ppm by weight or less in terms of chlorine.

Moreover, the hydrogen gas used in the reaction had a purity of 99.99 vol% and a water concentration of 0.5 ppm by weight or less. As for the other impurities, the concentration of sulfur compounds was 1 ppm by weight or less in terms of sulfur, the concentration of nitrogen compounds was 0.1 ppm by weight or less in terms of nitrogen, the concentration of oxygen compounds other than water was 0.1 ppm by weight or less in terms of oxygen, and the concentration of chlorine compounds was 0.1 ppm by weight or less in terms of chlorine.

### [Reaction method]

The solid acid catalyst was granulated to particles of 16 to 24 mesh. Four milliliters of the catalyst after being granulated was charged into a fixed-bed flow reactor with a length of 50 cm and an inner diameter of 1 cm and isomerization was performed after pre-treatment. Pre-treatment of the catalyst was performed at 200°C in a normal-pressure hydrogen gas current.

Isomerization of the hydrocarbons was performed at a reaction pressure (gauge pressure) of 3030 kPa (30 kg/cm²), an LHSV of 2 h⁻¹, a hydrogen/hydrocarbon ratio of 2 (mol/mol), and a reaction temperature of 150°C. The reaction was started using the reaction starting materials 1 to 4 as the starting materials and the gas discharged from the outlet of the reactor was analyzed by the gas chromatography after 200 hours from the initiation of the reaction. The composition at the outlet of the reaction tube, which is the analysis result, is shown in Table 1. It is clear that catalytic activity improves markedly when the water concentration by weight of the starting material was 1 ppm or less.

Moreover, the reaction was started at a reaction pressure (gauge pressure) of 30 kg/cm², LHSV of 2 h⁻¹, hydrogen/hydrocarbon ratio of 0.14 (mol/mol), and reaction temperature of 130°C using reaction starting material 5 as the starting material and the outlet gas of the reactor was analyzed by gas chromatography after 500 hours from the initiation of the reaction. The results are shown in Table 1.

**[Table 1]**

| Reaction starting material | Water concentration (ppm) | Composition at reaction tube outlet | |
|---|---|---|---|
| | | Isopentane/ pentane | 2,2-Dimethyl butane/hexane |
| Reaction starting material 1 | 40 | 0.506 | 0.056 |
| Reaction starting material 2 | 20 | 0.535 | 0.065 |
| Reaction starting material 3 | 0.9 | 0.760 | 0.329 |
| Reaction starting material 4 | 0.4 | 0.765 | 0.335 |
| Reaction starting material 5 | 0.1 | 0.759 | 0.330 |

### Example 2

The same solid acid catalyst as in Example 1 was used.

### [Reaction starting material]

Light naphtha was used as the starting hydrocarbon, which was a starting material for isomerization. This light naphtha comprised 4.9 wt% butane, 56.3 wt% pentane, 32.6 wt% hexane, 2.3 wt% cyclopentane, 2.8 wt% methyl cyclopentane and cyclohexane, and 1.1 wt% benzene. The light naptha had a water concentration of 40 ppm by weight and a sulfur concentration of 1 ppm by weight or less. Moreover, the hydrogen gas used as the starting hydrogen in the reaction had a purity of 99.99 vol% or higher and a water concentration of 0.5 ppm by weight or less.

### [Isomerization device]

The structure of the isomerization device is shown in Figure 1. The starting hydrocarbons were introduced from the starting material tank 1 by the pump 2, while the starting hydrogen was introduced from the hydrogen line 3, with these starting materials being mixed in the piping and introduced to the dehydration device 4. The starting material that had passed through the dehydration device 4 was introduced to the catalytic reactor 5 in which the above-mentioned solid acid catalyst had been charged. The reaction product in the catalytic reactor 5 was introduced to the gas-liquid separation device 6 and the hydrocarbon product was taken out from the lower bottom piping 7. The unreacted hydrogen that was separated by the gas-liquid separation device 6 was emitted from the upper piping 8, compressed by the compressor 9, mixed with the starting materials that had fed via the dehydration device 4, and once again introduced to the catalytic reactor 5. MOLECULAR SIEVES 3A had been packed in the dehydration device 4.

### [Reaction method]

The catalytic reactor 5 was a fixed-bed flow reactor with a length of 50 cm and an inner diameter of 1 cm and was heated from the outer periphery by an electric heater 10. Four milliliters of the solid acid catalyst that had been granulated to particles of 16 to 24 mesh was charged into the catalytic reactor 5 and isomerization was performed after pre-treatment. The pre-treatment of the catalyst was performed at 200°C in a normal-pressure hydrogen current.

Isomerization of the hydrocarbons was performed under conditions of a reaction pressure (gauge pressure) of 3,030 kPa (30 kg/cm²), LHSV of 2 h⁻¹, hydrogen/hydrocarbon ratio of 2 mol/mol, and reaction temperature of 150°C. Moreover, when the water concentration at the outlet of dehydration device 4 under these reaction conditions was analyzed with the Karl Fischer moisture meter, the water concentration was 0.4 ppm in terms of the weight of the water concentration after 200 hours from the initiation of the reaction. The composition of the hydrocarbons taken out from the lower piping 7 was analyzed by gas chromatography after 200 hours from the initiation of the reaction.

By way of comparison, reaction was performed without charging MOLECULAR SIEVES 3A into the dehydration device 4 served as reaction 1. On the other hand, the reaction in which MOLECULAR SIEVES 3A was charged into the dehydration device 4 was designated as reaction 2. The compositional proportions of hydrocarbons taken out from the lower piping 7, which are the gas chromatography analysis results, are shown in Table 2. The proportion of isopentane with five carbons in cyclic saturated hydrocarbons (isopentane/pentane), and the proportion of 2,2-dimethyl butane with six carbons in cyclic saturated hydrocarbons (2,2-dimethyl butane/hexane) were evaluated for the compositional proportions. It is clear that the results of the isomerization reaction are markedly improved by bringing the water concentration of the starting materials to 0.5 ppm or less with the dehydration device 4.

**[Table 2]**

| Reaction starting material | Compositional proportions at reaction tube outlet | |
|---|---|---|
| | Isopentane/ pentane | 2,2-Dimethyl butane/hexane |
| Reaction 1 | 0.504 | 0.054 |
| Reaction 2 | 0.763 | 0.332 |

### INDUSTRIAL APPLICABILITY

In accordance with the present invention, isomerization of hydrocarbons is performed by bringing starting hydrocarbons comprising a water concentration of 5 ppm or less, particularly to 1 ppm or less, in terms of the water weight, and a specific solid acid catalyst into contact with one another in the presence of hydrogen. The isomerization can be performed at a high efficiency without introducing a chlorine compound to the reaction system by using the specific solid acid catalyst.

Moreover, because it is not necessary to introduce a chlorine compound to the reaction system, the process whereby the chlorine compound is separated from the reaction product can be omitted and the problem of equipment corrosion by the chlorine compound can be avoided.

## Claims

1. A method of isomerization of hydrocarbons for obtaining isomerized hydrocarbons, comprising:
bringing a solid acid catalyst comprising a support which comprises portions made of zirconia and/or hydrated zirconia having a tetragonal crystal structure, and a sulfureous component supported in an amount of 1 to 10 wt%, in terms of the amount of sulfur in the solid acid catalyst, on the support into contact with starting hydrocarbons having a water concentration of 5 ppm or less in terms of the weight of the water in the presence of hydrogen and
performing isomerization of the hydrocarbons at a reaction temperature of 50 to 250°C and a reaction pressure range of 1,515-4,040 KPa (15 to 40 kgf/cm²).

2. A method of isomerization of hydrocarbons according to Claim 1, wherein the support in Claim 1 comprises portions made of zirconia and/or hydrated zirconia having a tetragonal crystal structure and portions made of alumina and/or hydrated alumina.

3. A method of isomerization of hydrocarbons according to Claim 1 or 2, wherein the solid acid catalyst described in Claim 1 comprises at least one metal component selected from the group of metals of Group 8, Group 9, and Group 10.

4. A method of isomerization of hydrocarbons according to any one of Claims 1 through 3, wherein the molar ratio of hydrogen/starting hydrocarbons in Claim 1 is not less than the amount of hydrogen necessary for saturation of an unsaturated component contained in the starting hydrocarbons and not greater than 10.

5. A method of isomerization of hydrocarbons according to any one of Claims 1 through 3, wherein the molar ratio of hydrogen/starting hydrocarbon in Claim 1 is from 0.1 to 10.

6. A hydrocarbon isomerization device, comprising:
a dehydration device, which reduces water concentrations of starting hydrogen and starting hydrocarbons;
a catalytic reactor, to which the starting hydrogen and the starting hydrocarbons that have passed through said dehydration device and recycled hydrogen are fed and in which a solid acid catalyst comprising a support which comprises portions made of zirconia and/or hydrated zirconia having a tetragonal crystal structure, and a sulfureous component supported in an amount of 1 to 10wt%, in terms of the amount of sulfur in the solid acid catalyst, on the support is charged;
a separation device, which separates a reaction product forwarded from the catalytic reactor into hydrogen to be recycled and a hydrocarbon product; and
a compressor, which compresses the recycled hydrogen forwarded from the separation device and feeds it to the catalytic reactor.

7. A hydrocarbon isomerization device according to Claim 6, wherein the support described in Claim 7 comprises portions made of zirconia and/or hydrated zirconia having a tetragonal crystals structure and portions made of alumina and/or hydrated alumina.

## Patentansprüche

1. Verfahren zur Isomerisation von Kohlenwasserstoffen zum Erhalt isomerisierter Kohlenwasserstoffe, umfassend:
das In-Kontakt-Bringen eines festen Säurekatalysators, umfassend einen Träger, der Teile aus Zirconiumdioxid und/oder hydratisiertem Zirconiumdioxid mit einer tetragonalen Kristallstruktur umfasst, und eine, bezogen auf die Menge an Schwefel im festen Säurekatalysator, in einer Menge von 1 bis 10 Gew.-% auf dem Träger geträgerte schwefelhaltige Komponente, mit Ausgangskohlenwasserstoffen mit einer Wasserkonzentration, bezogen auf das Gewicht des Wassers, von 5 ppm oder weniger in Gegenwart von Wasserstoff und
die Durchführung der Isomerisation der Kohlenwasserstoffe bei einer Reaktionstemperatur von 50 bis 250°C und einem Bereich des Reaktionsdrucks von 1.515 bis 4.040 kPa (15 bis 40 kgf/cm²).

2. Verfahren zur Isomerisation von Kohlenwasserstoffen gemäß Anspruch 1, bei dem der Träger in Anspruch 1 Teile aus Zirconiumdioxid und/oder hydratisiertem Zirkoniumdioxid mit einer tetragonalen Kristallstruktur und Teile aus Aluminiumoxid und/oder hydratisiertem Aluminiumoxid umfasst.

3. Verfahren zur Isomerisation von Kohlenwasserstoffen gemäß Anspruch 1 oder 2, bei dem der in Anspruch 1 beschriebene feste Säurekatalysator mindestens eine Metallkomponente, ausgewählt aus der Gruppe von Metallen der Gruppe 8, Gruppe 9 und Gruppe 10, umfasst.

4. Verfahren zur Isomerisation von Kohlenwasserstoffen nach einem der Ansprüche 1 bis 3, bei dem das Molverhältnis von Wasserstoff zu Ausgangskohlenwasserstoffen in Anspruch 1 nicht kleiner als die Wasserstoffmenge, die zur Sättigung einer im Ausgangswasserstoff enthaltenen Komponente notwendig ist, und nicht größer als 10 ist.

5. Verfahren zur Isomerisation von Kohlenwasserstoffen nach einem der Ansprüche 1 bis 3, bei dem das Molverhältnis von Wasserstoff zum Ausgangskohlenwasserstoff in Anspruch 1 0,1 zu 10 beträgt.

6. Kohlenwasserstoffisomerisationsvorrichtung, umfassend:
eine Dehydratationsvorrichtung, die die Wasserkonzentrationen von Ausgangswasserstoff und Ausgangskohlenwasserstoffen verringert;
einen katalytischen Reaktor, in den der Ausgangswasserstoff und die Ausgangskohlenwasserstoffe, die diese Dehydratationsvorrichtung passiert haben, und rückgeführter Wasserstoff eingespeist werden und der mit einem festen Säurekatalysator, umfassend einen Träger, der Teile aus Zirconiumdioxid und/oder hydratisiertem Zirconiumdioxid mit einer tetragonalen Kristallstruktur umfasst, und einer schwefelhaltige Komponente, die, bezogen auf die Schwefelmenge im festen Säurekatalysator in einer Menge von 1 bis 10 Gew.-% auf dem Träger geträgert ist, beschickt wird,
eine Trennvorrichtung, die ein aus dem katalytischen Reaktor ausgetragenes Reaktionsprodukt in Wasserstoff zur Rückführung und ein Kohlenwasserstoffprodukt trennt; und
einen Kompressor, der den aus der Trennvorrichtung ausgetragenen rückgeführten Wasserstoff komprimiert und diesen in den katalytischen Reaktor einspeist.

7. Kohlenwasserstoffisomerisationsvorrichtung nach Anspruch 6, wobei der in Anspruch 6 beschriebene Träger Teile aus Zirconiumdioxid und/oder hydratisiertem Zirconiumdioxid mit einer tetragonalen Kristallstruktur und Teile aus Aluminiumoxid und/oder hydratisiertem Aluminiumoxid umfasst.

## Revendications

1. Procédé d'isomérisation d'hydrocarbures pour obtenir des hydrocarbures isomérisés, comprenant :
l'apport d'un catalyseur acide solide comprenant un support qui comprend des parties faites d'oxyde de zirconium et/ou d'oxyde de zirconium hydraté ayant une structure cristalline quadratique, et un composant sulfureux déposé en une quantité de 1 à 10 % en poids, en termes de quantité de soufre du catalyseur acide solide, sur le support en contact avec les hydrocarbures de départ ayant une concentration en eau de 5 ppm ou moins en poids d'eau, en présence d'hydrogène et
la réalisation de l'isomérisation des hydrocarbures à une température de réaction de 50 à 250 °C et une gamme de pressions de réaction de 1 515 à 4 040 kPa (15 à 40 kgf/cm²).

2. Procédé d'isomérisation d'hydrocarbures selon la revendication 1, dans lequel le support de la revendication 1 comprend des parties faites d'oxyde de zirconium et/ou d'oxyde de zirconium hydraté ayant une structure cristalline quadratique et des parties faites d'alumine et/ou d'alumine hydratée.

3. Procédé d'isomérisation d'hydrocarbures selon la revendication 1 ou 2, dans lequel le catalyseur acide solide décrit dans la revendication 1 comprend au moins un composant métallique choisi dans le groupe des métaux du groupe 8, du groupe 9 et du groupe 10.

4. Procédé d'isomérisation d'hydrocarbures selon l'une quelconque des revendications 1 à 3, dans lequel le rapport molaire hydrogène/hydrocarbures de départ dans la revendication 1 n'est pas inférieur à la quantité d'hydrogène nécessaire à la saturation d'un composant insaturé contenu dans les hydrocarbures de départ et non supérieur à 10.

5. Procédé d'isomérisation d'hydrocarbures selon l'une quelconque des revendications 1 à 3, dans lequel le rapport molaire hydrogène/hydrocarbures de départ dans la revendication 1 est de 0,1 à 10.

6. Dispositif d'isomérisation d'hydrocarbures, comprenant :
un dispositif de déshydratation, qui réduit les concentrations en eau de l'hydrogène de départ et des hydrocarbures de départ ;
un réacteur catalytique, dans lequel on charge l'hydrogène de départ et les hydrocarbures de départ qui ont traversé ledit dispositif de déshydratation et l'hydrogène recyclé et dans lequel on charge le catalyseur acide solide comprenant un support qui comprend des parties faites d'oxyde de zirconium et/ou d'oxyde de zirconium hydraté ayant une structure cristalline quadratique, et un composant sulfureux déposé en une quantité de 1 à 10 % en poids, en termes de la quantité de soufre dans le catalyseur acide solide sur le support ;
un dispositif de séparation, qui sépare le produit de réaction provenant du réacteur catalytique en hydrogène à recycler et un produit hydrocarboné ; et
un compresseur, qui comprime l'hydrogène recyclé provenant du dispositif de séparation et le charge dans le réacteur catalytique.

7. Dispositif d'isomérisation d'hydrocarbures selon la revendication 6, dans lequel le support décrit à la revendication 6 comprend des parties faites d'oxyde de zirconium et/ou d'oxyde de zirconium hydraté ayant une structure cristalline quadratique et des parties faites d'alumine et/ou d'alumine hydratée.
